(19) Europäisches Patentamt
European Patent Office
Office européen des brevets

(11) EP 0 497 659 B1

(12) **FASCICULE DE BREVET EUROPEEN**

(45) Date de publication et mention de la délivrance du brevet: **10.04.1996 Bulletin 1996/15**

(51) Int. Cl.[6]: **C07D 403/06**, C07D 403/14, C07D 417/14, C07D 471/14, C07D 403/12

(21) Numéro de dépôt: **92400164.7**

(22) Date de dépôt: **22.01.1992**

(54) **Dérivés d'aryl (ou hétéroaryl)-piperaz inyl-alkyl-azoles, leur préparation et leur application en tant que médicaments**

Aryl(oder Heteroaryl)-piperazinyl-alkyl-azol-derivate, ihre Herstellung und ihre Anwendung als Medikament

Aryl(or Heteroaryl)-piperazinyl-alkyl-azole derivatives, their preparation and their application as drugs

(84) Etats contractants désignés:
**AT BE CH DE DK FR GB GR IT LI LU NL PT SE**

(30) Priorité: **28.01.1991 FR 9100923**

(43) Date de publication de la demande:
**05.08.1992 Bulletin 1992/32**

(73) Titulaire: **LABORATORIOS DEL DR. ESTEVE, S.A.**
**E-08026 Barcelona (ES)**

(72) Inventeurs:
- **Vidal, Ramon Mercé**
  **E-08014 Barcelone (ES)**
- **Constansa, Jordi Frigola**
  **E-08013 Barcelone (ES)**
- **Corominas, Juan Parés**
  **E-08025 Barcelone (ES)**

(74) Mandataire: **Ahner, Francis et al**
**CABINET REGIMBEAU**
**26, avenue Kléber**
**F-75116 Paris (FR)**

(56) Documents cités:
**EP-A- 0 056 866**
**EP-A- 0 241 053**
**EP-A- 0 379 990**
**EP-A- 0 382 637**
**EP-A- 0 407 844**
**DE-A- 2 143 730**
**US-A- 3 362 956**
**US-A- 3 472 854**
**US-A- 3 491 098**
**US-A- 3 511 841**
**US-A- 3 562 278**

- **CHEM. SOC. REVIEWS vol. 18, 1979, pages 563-580; C.W. THORNBER: "Isosterism and molecular modification in drug design"**
- **The Journal of Pharmacology and Experimental Therapeutics, vol. 262(1), 90-98**

Remarques:
Le dossier contient des informations techniques présentées postérieurement au dépôt de la demande et ne figurant pas dans le présent fascicule.

EP 0 497 659 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention se rapporte à des nouveaux dérivés de 1-{4-[4-(2-aryl (ou hétéroaryl))-1-pipérazinyl]-alkyl}-1*H*-azole, leur procédé de préparation, ainsi que leur application en tant que médicaments.

Les composés objet de la présente invention peuvent être utilisés dans l'industrie pharmaceutique comme intermédiaires et pour la préparation de médicaments.

On connaissait dejà des 1-{4-[4-(aryl (ou hétéroaryl))-1-pipérazinyl]-alkyl}-N-hétérociclyl-diones, par exemple:

Wu Y.H. et al., J. Med. Chem. 1969, 12, 876; Wu Y.H. et al., J. Med. Chem. 1972, 15, 477; Temple D.L. et al., U.S.Pat 4.456.756; Yevich J.P. et al., J. Med. Chem. 1983. 26, 194; mais par contre, avec des azoles on trouve seulement nos propres travaux (Brevet français, A. Colombo et al., FR 2642759).

Nous avons maintenant découvert que les nouveaux dérivés de 1-{4-[4-(2-aryl (ou hétéroaryl))-1-pipérazinyl]-alkyl}-1*H*-azole, ainsi que leurs sels physiologiquement acceptables qui font l'objet de la présente invention, présentent une activité pharmacologique sur le système nerveux central, en particulier ils présentent des activités anxiolytique et tranquillisante, ainsi qu'antidépresive, dans l'inhibition du syndrome d'abstinence et dans les troubles associés à la cognition tels que la démence sénile, les disfonctions de la mémoire, la détérioration de la conscience, etc.; et sur le système cardiovasculaire en particulier une activité antihypertensive. Les nouveaux composés, qui font l'objet de la présente invention, peuvent être utilisés en thérapeutique dans le traitement de l'anxiété, de la depréssion, du syndrome d'abstinence, des troubles de la cognition et de l'hypertension.

Les composés objet de la présente invention répondent à la formule générale I

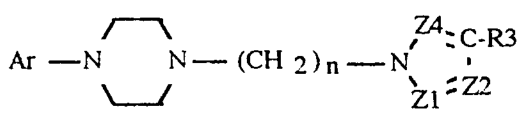

dans laquelle

- Ar représente un radical aromatique azoté ou non, choisi parmi les groupes phényle, phényle substitué par un radical méthoxy, 2-pyrimidine, 2-N-méthylimidazol et 3-(1,2-benzisothiazole),
- n peut prendre les valeurs 2 à 7,
- $Z_1$ représente un atome d'azote ou un groupement C-$R_1$,
- $Z_2$ représente un atome d'azote ou un groupement C-$R_2$,
- $Z_4$ représente un atome d'azote ou un groupement C-$R_4$,

et

$R_1$, $R_2$, $R_3$ et $R_4$, identiques ou différents pouvant également former une partie d'un autre cycle, aromatique ou non, représentent un atome d'hydrogène, un halogène, un radical alkyle inférieur, un radical carboxamido, un radical alkyloxycarbonyle, ou un radical phényle,

- à la condition que lorsque Ar représente le groupe 2-pyrimidine, le radical hétéroaromatique

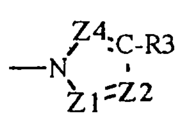

est choisi parmi le groupe comprenant les radicaux imidazolyle trisubstitués pour lesquels

Z1 représente un groupement C-R1,
Z2 représente un atome d'azote,
Z4 représente un groupement C-R4, et

R1, R3 et R4, identiques ou différents, sont différents d'un atome d'hydrogène,
et les radicaux benzimidazolyle substitués ou non, pour lesquels

Z1 représente un groupement C-R1,

Z2 représente un atome d'azote,

Z4 représente un groupement C-R4, et

R3 et R4 représentent un radical -CH=CH-CH=CH-et leurs sels d'addition des acides pharmaceutiquement acceptables,

- à l'exception des composés pour lesquels Ar représente un groupe phényle, substitué ou non, $Z_1$ représente un groupement C-$R_1$, $Z_2$ représente un atome d'azote, $Z_4$ représente un groupement C-$R_4$, et $R_3$-$R_4$ forment une partie d'un autre cycle aromatique, et
- à l'exception des composés pour lesquels Ar représente un groupe phényle, substitué ou non, et le radical hétéroaromatique

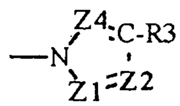

représente un groupe 4, 5, 6 ou 7-azaindolyle.

Des dérivés de phényl-pipérazinyl-alkyl-(4, 5, 6 ou 7)-azaindole sont notamment décrits dans le brevet US-A- 3 511 841.

Les nouveaux dérivés de formule générale I peuvent être préparés, conformément à l'invention, selon l'une quelconque des méthodes suivantes.

**METHODE A**

Par réaction d'un composé de formule générale II

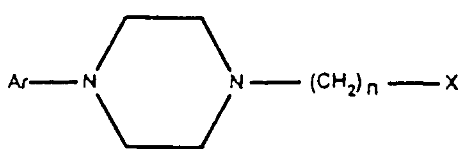

II)

dans laquelle

Ar et n ont les significations mentionnées précédemment et X représente un atome d'halogène, ou un groupe partant choisi parmi le tosyloxy ou le mésiloxy, avec un composé de formule générale III

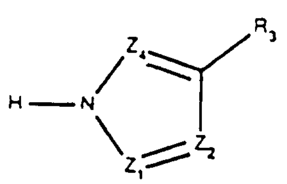

(III)

dans laquelle

$Z_1$, $Z_2$, $Z_4$ et $R_3$ ont les significations mentionnées précédemment.

La réaction s'effectue en présence d'un solvant adéquat, par exemple le diméthylsulfoxyde, la diméthylformamide, un alcool, un hydrocarbure, aromatique ou non, un éther, tel le dioxanne ou l'éther diphénylique, ou un mélange de ces solvants. Cette réaction est aventageusement conduite en présence d'une base telle les hydroxydes. les carbonates ou les bicarbonates des métaux alcalins, ou bien un mélange de ces bases.

Les températures les plus adéquates varient entre la température ambiante et la température de reflux du solvant, et le temps réactionnel est compris entre 1 heure et 24 heures.

METHODE B

On suit la méthode A mais la réaction conduisant à un mélange d'isomères, on réalise en fin de réaction une séparation des composants par des méthodes physiques, telles la distillation, les cristallisations ou les méthodes chromatographiques conventionneles.

METHODE C

Par réaction d'un composé de formule générale IV

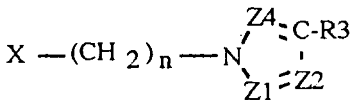

(IV)

dans laquelle

$Z_1$, $Z_2$, $Z_4$, $R_3$, n et X ont les significations mentionnées précédemment, avec un composé de formule générale V

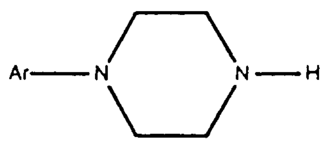

(V)

dans laquelle

Ar a les significations mentionnées précédemment.

La réaction s'effectue en présence d'un solvant adéquat, par exemple le diméthylsulfoxyde, la diméthylformamide, un alcool, un hydrocarbure, aromatique ou non, un éther, tel le dioxanne ou l'éther diphénylique, ou un mélange de ces solvants. Cette réaction est aventageusement conduite en présence d'une base telle les hydroxydes, les carbonates ou les bicarbonates des métaux alcalins, ou bien un mélange de ces bases.

Les températures les plus adéquates varient entre la température ambiante et la température de reflux du solvant, et le temps réactionnel est compris entre 1 heure et 24 heures.

METHODE D

Par réaction d'un composé de formule générale VI

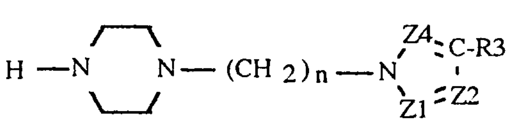

(VI)

dans laquelle

$Z_1$, $Z_2$, $Z_4$, $R_3$ et n ont les significations mentionnées précédemment, avec un composé de formule générale VII

Ar—X (VII)

dans laquelle

Ar et X ont les significations mentionnées précédemment.

La réaction s'effectue en présence d'un solvant adéquat, par exemple le diméthylsulfoxyde, la diméthylformamide, un alcool, un hydrocarbure, aromatique ou non, un éther, tel le dioxanne ou l'éther diphénylique, ou un mélange de ces

solvants. Cette réaction est aventageusement conduite en présence d'une base telle les hydroxydes, les carbonates ou les bicarbonates des métaux alcalins, ou bien un mélange de ces bases.

Les températures les plus adéquates varient entre la température ambiante et la température de reflux du solvant, et le temps réactionnel est compris entre 1 heure et 24 heures.

METHODE E

Par réaction d'un composé de formule générale VIII

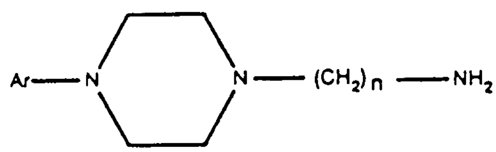

(VIII)

dans laquelle

Ar et n ont les significations mentionnées précédemment, avec le 2,5-diméthoxytétrahydrofurane.

La réaction s'effectue en présence d'un solvant adéquat, par exemple le diméthylsulfoxyde, la diméthylformamide, la diméthylacetamide ou l'acide acètique.

Les températures les plus adéquates varient entre 60°C et 120°C et le temps réactionnel est compris entre 5 minutes et 3 heures.

METHODE F

Par hydrolyse d'un composé de formule générale I dans laquelle Ar, n, $Z_1$, $Z_2$, $Z_4$, et $R_3$ ont les significations mentionnées précédemment et où au moins l'un des substituants $R_1$, $R_2$, $R_3$ et $R_4$ représente un groupement carboxylate d'alkyle.

Cette réaction peut s'effectuer tant en milieu basique, de préférence avec le concours d'une base alcaline telle que l'hydroxyde de sodium ou l'hydroxyde de potassium, à des températures comprises entre 40°C et 100°C, qu'acide, de préférence avec l'acide chorhydrique, l'acide sulfurique, l'acide p-toluènesulfonique, à des temperatures comprises entre 40°C et 100°C.

METHODE G

Par réaction d'un composé de formule générale I dans laquelle Ar, n, $Z_1$, $Z_2$, $Z_4$, et $R_3$ ont les significations mentionnées précédemment mais où l'un au moins des substituants $R_1$, $R_2$, $R_3$ et $R_4$ représente un radical carboxylique, avec un radical amino ou un radical alkylamino, plus particulièrement l'ammoniac. Cette reaction peut avoir lieu par des méthodes dejà connus dans la littérature tels que l'anhydride mixte ou l'halure d'acide entre autres.

La condensation finale avec l'amine s'effectue sans solvant ou en présence d'un solvant adéquat, par exemple: dans un hydrocarbure aromatique tel que le benzène ou le toluène, d'un composé chloré tel que le chlorure de méthylène ou le chloroforme, une cétone, un éther ou une amide telle que la diméthylformamide ou bien des mélanges de ces solvants. De plus, la présence d'une base inorganique telle que le carbonate de sodium ou organique telle que la pyridine ou la triéthylamine, est souhaitable.

Les températures appropriées oscillent entre -10°C et la température d'ébullition du solvant et le temps réactionnel est compris entre 1 heure et 24 heures

METHODE H

Par réduction d'un composé de formule générale IX, preparé par une méthode tout à fait semblable à celle de la méthode C,

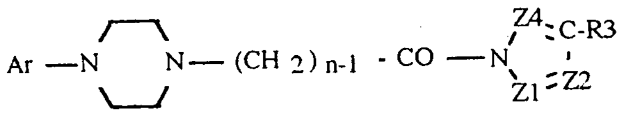

(IX)

dans laquelle

Ar, $Z_1$, $Z_2$, $Z_4$, $R_3$ et n ont les significations mentionnées précédemment.

La réaction s'effectue de préférence avec le concours d'un hydrure métallique tel que l'hydrure d'aluminium et de lithium dans un solvant adéquat par exemple un éther tel que le diéthyléther ou le tétrahydrofuranne. La température se mantient entre 25° et 40°C pendant l'addition, et entre la température ambiante et la température de reflux du solvant pendant la réaction. Le temps réactionnel est compris entre 1 heure et 24 heures.

METHODE I

Par réaction d'un composé basique de formule générale I avec un acide minéral ou organique non toxique dans le sein d'un solvant approprié, qui peut être par exemple un alcool tel que le méthanol, l'éthanol ou l'un quelconque des propanols ou des butanols, un ester tel que l'acétate d'éthyle ou un nitrile tel que l'acétonitrile; et après les techniques habituelles de précipitation, cristallisation, etc. on obtient le sel correspondant.

L'acide minéral est choisi, entre d'autres, parmi l'acide chlorhydrique, sulfurique, phosphorique et les sels métalliques d'acide tel que l'hydrogène sulfate de potassium; et l'acide organique choisi parmi les mono, di ou tricarboxyliques, par exemple les acides: acétique, lactique, malonique, succinique, glutarique, fumarique, malique, tartarique, citrique, ascorbique, maleique, benzoique, phenylacétique, cinnamique, salicilique et des acides sulfoniques. On peut former le mono ou di-sel d'acide et ces sels peuvent être en forme hydratée ou non.

Dans les exemples suivants, on indique la préparation de nouveaux dérivés selon l'invention. On décrira également quelques formes d'emploi.

Les exemples ci-après, donnés à simple titre d'illustration, en particulier les exemples illustrant les méthodes B, D, E, F, G et H, selon l'invention, qui décrivent la préparation de dérivés exclus de la définition de la formule générale I, mais de structure voisine. Les dites méthodes ne font qu'illustrer un mode opératoire d'utilité quand on contemple la préparation de composés hétérocycliques en accord avec l'invention.

METHODE A

Exemple 4

Préparation de 1-{4-[4-(2-pyrimidinyl)-1-pipérazinyl]-butyl}-1*H*-benzimidazole.

On chauffe à reflux pendant 14 heures un mélange de 6 g (20 mmoles) de 2-[4-(4-bromobutyl)-1-pipérazinyl]-pyrimidine, 2,36 g (20 mmoles) de benzimidazole et 4,1 g (30 mmoles) de carbonate de potassium, dans 60 ml de diméthylformamide. On évapore sous vide, on ajoute du chloroforme, on lave à l'eau , on sèche sur sulfate de sodium, on évapore sous vide et on obtient 4,8 g de 1-{4-[4-(2-pyrimidinyl)-1-pipérazinyl]-butyl}-1*H*-benzimidazole, qu'on peut recristalliser dans de l'éther éthylique, avec un point de fusion de 85-88°C.

Les composés identifiés par les exemples 1 à 4, 8 à 14, 19 à 24, 26 et 27 sont obtenus par la même procédure et les données pour leur identification sont exposées dans les tableaux I, II et III.

METHODE B

Préparation de 1-{4-[4-(2-pyrimidinyl)-1-pipérazinyl]-butyl}-3*H*-imidazo [5,4-b]-pyridine et 1-{4-[4-(2-pyrimidinyl)-1-pipérazinyl]-butyl}-1*H*-imidazo[4,5-b]-pyridine.

On suit la méthode A mais avec du 1*H*-imidazo[4,5-b]-pyridine comme produit de départ.

On obtient ainsi un mélange de ces deux composants, qu'on sépare par chromatographie préparative à haute pression.

Les composés identifiés par les exemples 6 et 7 sont obtenus par une méthode similaire et les données pour leur identification sont exposées dans le tableau I.

METHODE C

Exemple 18

Préparation de 4-chloro-1-[4-(4-phényl-1-pipérazinyl)-butyl]-1*H*-pyrazole.

On chauffe à reflux pendant 24 heures a mélange de 3,56 g (15 mmoles) de N-(4-bromobutyl)-4-chloropyrazole, 2,43 g (15 mmoles) de 1-phénylpipérazine et 2,76 g (20 mmoles) de carbonate de potassium dans 50 ml de diméthylformamide. On évapore sous vide, on ajoute du chloroforme, on lave à l'eau, on sèche sur sulfate de sodium, on évapore sous vide et on obtient 3,1 g de 4-chloro-1-[4-(4-phényl-1-pipérazinyl)-butyl]-1H-pyrazole, qu'on peut recristaliser dans l'éther éthylique avec un point de fusion de 58-61 °C.

Les données spectroscopiques pour l'identification de ce produit sont exposées dans le tableau II.

METHODE D

Préparation de 1-{4-[4-(2-pyrimidinyl)-1-pipérazinyl]-butyl}-pyrrole.

On chauffe à réflux pendant 5 heures un mélange de 4,14 g (20 mmoles) de 1-[4-(pipérazinyl)-butyl]-pyrrole, 2,29 g (20 mmoles) de 2-chloropyrimidine et 4,1 g (30 mmoles) de carbonate de potassium, dans 60 ml de diméthylformamide. On évapore sous vide, on ajoute du chloroforme, on lave à l'eau,on sèche sur sulfate de sodium, on évapore sous vide et on obtient 3,3 g de 1-{4-[4-(2-pyrimidinyl)-1-pipérazinyl]-butyl}-pyrrole, sous une forme liquide.

METHODE E

Préparation de 1-{4-[4-(2-pyrimidinyl)-1-pipérazinyl]-butyl}-pyrrole.

On chauffe à reflux pendant 1 heure un mélange de 4 g (17 mmoles) de 2-[4-(4-aminobutyl)-1-pipérazinyl]-pyrimidine et 3,3 g de 2,5-diméthoxytétrahydrofurane dans 10 ml d'acide acétique. On verse sur l'eau froide, on neutralise avec de la soude 10%, on extrait du chloroforme, on sèche, on évapore et on obtient 2,2 g de 1-{4-[4-(2-pyrmidinyl)-1-pipérazinyl]-butyl}-pyrrole.

Les produits des exemples 15 à 17 sont obtenus par la même procédure.

Les données spectroscopiques pour l'identification de ces produits sont exposées dans les tableaux I et II.

METHODE F

Préparation de 4-carboxy-1-{4-[4-(2-pyrimidinyl)-1-pipérazinyl]-butyl}-1*H*-pyrazole.

On chauffe à reflux pendant 4 heures un mélange de 4,4 g (12,2 mmoles) de 4-carboxylate d'éthyle-1-{4-[4-(2-pyrimidinyl)-1-pipérazinyl]-butyl}-1*H*-pyrazole dans 50 ml de HCl, 2 N. On refroidit avec un bain de glace, on neutralise avec de l'ammoniac et on extrait avec du chloroforme. On obtient ainsi 3,4 g de 4-carboxy-1-{4-[4-(2-pyrimidinyl)-1-pipérazinyl]-butyl}-1*H*-pyrazole, avec un point de fusion de 104-105°C.

METHODE G

Préparation de 4-carboxamido-1-{4-[4-(2-pyrimidinyl)-1-pipérazinyl]-butyl}-1*H*-pyrazole.

On ajoute lentement 1,1 g (10,3 mmoles) de chloroformiate d'éthyle sur une solution refroidie à 0°C 3,4 g (10,3 mmoles) de 4-carboxy-1-{4-[4-(2-pyrimidinyl)-1-pipérazinyl]-butyl}-1*H*-pyrazole,(exemple 6) et 1,04 g (10,3 mmoles) de triéthylamine dans 90 ml de diméthylformamide. Après 30 minutes on passe un courant d'ammoniac sec, on laisse en agitation pendant 1 heure à 0°C, on abandonne à température ambiante pendant 2 heures, on filtre, on lave avec de la diméthylformamide, on sèche, on évapore et on obtient 1,5 g de 4-carboxamido-1- 4-[-(2-pyrimidinyl)-1-pipérazinyl]-butyl -1H-pyrazole, qu'on peut recristalliser dans l'acetone, avec un point de fusion de 124°C.

Les données spectroscopiques pour l'identification de ce produit sont exposées dans le tableau I.

METHODE H

Préparation de 1,3-diméthyl-5-{3-[4-(2-méthoxyphényl)-1-pipérazinyl]-propylamino}-1H-pyrazole.

On met en suspension 0,5g (13 mmoles) de tétrahydrure d'aluminium et de lithium dans 100 ml de tétrahydrofuranne et on ajoute goutte à goutte pendant 30 minutes une solution de 3,9 g (11 mmoles) de 1,3-diméthyl-5-{3-[4-(2-méthoxyphényl)-1-pipérazinyl]-propionamido}-1*H*-pyrazole, dans 50 ml de tétrahydrofuranne. On maintient à température ambiante pendant l'addition et ensuite on chauffe à reflux pendant 3 heures. On détruit l'excès d'hydrure d'aluminium et de lithium, on filtre la fraction minérale insoluble, on élimine le tétrahydrofuranne, on dissout avec du chloroforme, on lave à l'eau, on sèche avec du sulfate de sodium anhydre, on évapore à sec et on obtient 3 g (81%) de 1,3-diméthyl-5-{3-[4-(2-méthoxyphényl)-1-pipérazinyl]-propylamino}-1*H*-pyrazole.

METHODE I

Exemple 25

Préparation du citrate de 4,5-dichloro-2-méthyl-1-{4-[4-(2-pyrimidinyl)-1-pipérazinyl]-butyl}-1*H*-imidazole.

On dissout 5g de 4,5-dichloro-2-méthyl-1-{4-[4-(2-pyrimidinyl)-1-pipérazinyl]-butyl}-1*H*-imidazole, (exemple 11), dans 50 ml d'alcool éthylique et on ajoute une solution de 3,15 g d'acide citrique monohydrate dans 20 ml d'éthanol. On agite pendant 1 heure et on laisse cristalliser à la température ambiante. On obtient 7,1 g de cristaux de point de fusion 137-138°C qui correspondent au citrate de 4,5-dichloro-2-méthyl-1-{4-[4-(2-pyrimidinyl)-1-pipérazinyl]-butyl}-1*H*-imidazole.

Les données spectroscopiques por l'identification de ce produit sont exposées dans le tableau III.

TABLEAU I

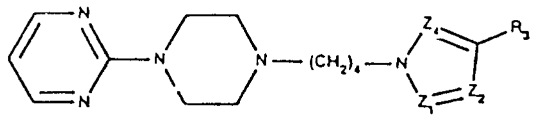

| Exemple | $Z_1$ | $Z_2$ | $Z_4$ | $R_3$ | P.F. | IR $cm^{-1}$ | RMN Solvant | $^1H$-RMN(100 MHz),δ,J-Hz |
|---|---|---|---|---|---|---|---|---|
| 1 | CPh | N | CPh | Ph | Huile | 2942, 1585, 1546, 1501, 1445, 1360, 1260, 983, 698 (film) | $CDCl_3$ | 1,55(m,4H); 1,95-2,33 (a.c. 6H); 3,69-4,07(a.c. 6H) 6,47(t,J=4,7Hz,1H); 7,13-7,67 (a.c. 15,H); 8,26 (d,J=4,7Hz,2H) |
| 2 | CMe | N | CPh | Ph | Huile | 2942, 1585, 1547, 1500, 1446, 1393, 1260, 983, 760, 698 (film) | $CDCl_3$ | 1,43(m,4H); 2,18-2,47 (a.c. 9H); 3,72-3,76(a.c. 6H) 6,47(t,J=4,7Hz,1H); 7,09-7,39 (a.c. 10H); 8,26 (d,J=4,7Hz,2H) |
| 3 | CMe | N | CCl | Cl | Huile | 2942, 1586, 1547, 1500, 1447, 1359, 1259, 1245, 983 (film) | $CDCl_3$ | 1,45-1,84(a.c. 4H); 2,26-2,57 (a.c. 9H); 3,74-4,05(a.c. 6H) 6,48(t,J=4,7Hz,1H); 8,30 (d,J=4,7Hz,2H) |

TABLEAU I (suite)

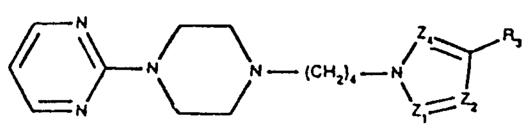

| Exemple | $Z_1$ | $Z_2$ | $Z_4$ | $R_3$ | P.F. | IR $cm^{-1}$ | RMN Solvant | $^1H$-RMN(100 MHz),δ,J-Hz |
|---|---|---|---|---|---|---|---|---|
| 4 | CH | N | C-CH=CH-CH=CH- | | 85-88°C | 2944, 1581, 1542, 1488, 1466, 1355, 1259, 741 (KBr) | DMSO-$d_6$ | 1,40(m,2H); 1,82(m,2H); 2,26-2,42(a.c. 6H); 3,62-3,71 (a.c. 4H); 4,24(t-J=6,9Hz,2H) 6,56(t,J=4,7Hz,1H); 7,16-7,26 a.c. 2H); 7,55-7,70(a.c. 2H); 8,22-8,34(a.c. 3H) |
| 5 | CCl | N | C-CH=CH-CH=CH- | | 153-145°C | 2940, 1583, 1542, 1491, 1466, 1443, 1383, 1264, 1128, 981, 742 (KBr) | DMSO-$d_6$ | 1,50(m,2H); 1,81(m,2H); 2,20-2,42(a.c. 6H); 3,67 (m,4H); 4,28(t,J=7Hz,2H); 6,58(t,J=4,7Hz,1H); 7,30 (m,2H); 7,60(m,2H); 8,31 (d,J=4,7Hz,2H) |

TABLEAU I (suite)

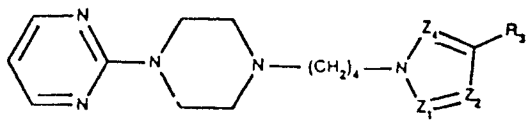

| Exemple | $Z_1$ | $Z_4$ | $R_3$ | $Z_2$ | P.F. | IR $cm^{-1}$ | RMN Solvant | $^1$H-RMN(100 MHz),δ,J-Hz |
|---|---|---|---|---|---|---|---|---|
| 6 | CH | Cl<br>C-CH=CH-C=CH- | | N | 82-84°C | 2945, 1583, 1544, 1492, 1356, 1260, 983, 799 (KBr) | $CDCl_3$ | 1,55(m,2H); 1,94(m,2H); 2,30-2,48(a.c. 6H); 3,75-3,85 (a.c. 4H); 4,16(t,J=7Hz,2H); 6,45(t,J=4,7Hz,1H); 7,27 (s,1H); 7,34(dd,J=9Hz,J'=2Hz, 1H); 7,70(d,J=9Hz); 7,87 (d,J=2Hz,1H); 8,27 (d,J=4,7Hz,2H) |
| 7 | CH | Cl<br>C-CH=C-CH=CH- | | N | 91-93°C | 2945, 1585, 1543, 1490, 1350, 1260, 983, 799 (KBr) | $CDCl_3$ | 1,55(m,2H); 1,94(m,2H); 2,30-2,48(a.c. 6H); 3,75-3,85 (a.c. 4H); 4,16(t,J=7Hz,2H); 6,45(t,J=4,7Hz,1H); 7,16 (dd,J=9Hz,J'=2Hz,1H); 7,27 s,1H); 7,83 (d,J=9Hz,1H); 7,87(d,J=2Hz,1H); 8,27 (d,J=4,7Hz,2H) |
| 8 | CMe | C-CH=CH-CH=CH- | | N | 101-102°C | 2938, 2820, 1583, 1542, 1494, 1405, 1357, 1258, 983, 798, 744 (KBr) | $CDCl_3$ | 1,56-1,93(a.c. 4H); 2,30-2,47 (a.c. 6H); 2,58(s,3H); 3,79 (t,J=5,2Hz,4H); 4,10 (t,J=7,3Hz,2H); 6,43 (t,J=4,7Hz,1H); 7,22(m,3H); 7,67(m,1H); 8,26 (d,J=4,7Hz,2H) |
| 9 | CH | $CH_3$ $CH_3$<br>C-CH=C—C=CH- | | N | 105-106°C | 2946, 1584, 1542, 1491, 1466, 1362, 1262, 983, 800, 742 (KBr) | $CDCl_3$ | 1,50(m,2H); 1,85(m,2H); 2,25-2,43(a.c. 12H); 3,76 (t,J=5,0Hz,4H); 4,07 (t,J=7,0Hz,2H); 6,40 (t,J=4,7Hz,1H); 7,11(s,1H); 7,51(s,1H); 7,71(s,1H); 8,23 (d,J=4,7Hz,2H) |

TABLEAU II

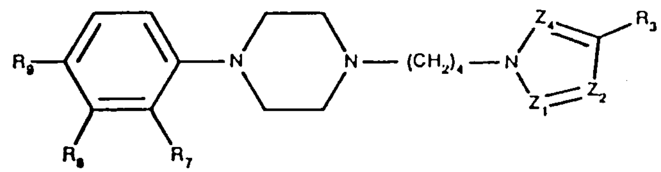

| Exemple | $Z_1$ | $Z_2$ | $R_3$ | $Z_4$ | $R_7$ | $R_8$ | $R_9$ | P.F. | IR $cm^{-1}$ | RMN Solvant | $^1$H-RMN(100 MHz), $\delta$, J=Hz |
|---|---|---|---|---|---|---|---|---|---|---|---|
| 10 | N | CH | Cl | CH | H | H | MeO- | 76-77°C | 2833, 1511, 1448, 1247, 1029, 979, 824 (KBr) | DMSO-$d_6$ | 1,43(m,2H); 1,78(m,2H); 1,71-2,48(a.c. 6H); 2,93-3,02 (m,4H); 3,67(s,3H); 4,09 t,J=6,8Hz,2H); 6,83(s,4H); 7,52(s,1H); 7,98(s,1H) |
| 11 | CMe | N | Cl | CCl | H | H | MeO- | 73-75°C | 2940, 2818, 1512, 1457, 1245, 1183, 1036, 826 (KBr) | DMSO-$d_6$ | 1,33-1,87(a.c. 4H); 2,32 (s,3H); 2,41-2,51(a.c. 6H); 2,82-3,0(m,4H); 3,67(s,3H); 3,93(t,J=7,2Hz,2H); 6,83 (s,4H) |
| 12 | N | CH | Cl | CH | MeO- | H | H | Huile | 2941, 2816, 1500, 1450, 1241, 749, (film) | DMSO-$d_6$ | 1,39(m,2H); 1,77(m,2H); 2,22-2,45(a.c. 6H); 2,92 (m,4H); 3,76(s,3H); 4,07 (t,J=6,0Hz,2H); 6,87(m,4H); 7,51(s,1H); 7,95(s,1H) |
| 13 | CMe | N | Cl | CCl | MeO- | H | H | 82-83°C | 2943, 2820, 1502, 1405, 1241, 1030, 746 (KBr) | DMSO-$d_6$ | 1,43-1,60(a..c. 4H); 2,33 (s,3H); 2,40-2,50(a.c. 6H); 2,95(m,4H); 3,76(s,3H); 3,93(t,J=7,0Hz,2H); 6,89 (m,4H) |
| 14 | N | CH | Cl | CH | H | MeO- | H | Huile | 2943, 2820, 1601, 1578, 1496, 1451, 1203, 1171, 970 (film) | $CDCl_3$ | 1,52(m,2H); 1,85(m,2H); 2,28-2,56(a.c. 6H); 3,16 (m,4H); 3,7(s,3H); 4,05 (t,J=7,0Hz,2H); 6,4(m,3H); 7,15(m,1H); 7,34(s,1H); 7,40(s,1H) |

TABLEAU II (suite)

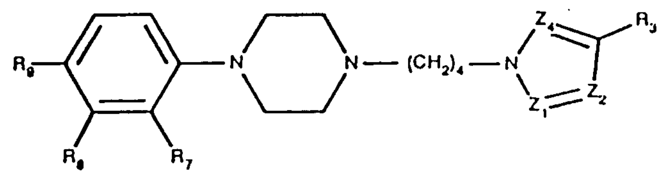

| Exemple | $Z_1$ | $Z_2$ | $R_3$ | $Z_4$ | $R_7$ | $R_8$ | $R_9$ | P.F. | IR $cm^{-1}$ | RMN Solvant | $^1$H-RMN(100 MHz), $\delta$, J=Hz |
|---|---|---|---|---|---|---|---|---|---|---|---|
| 15 | CH | CH | H | CH | H | H | MeO- | Huile | 2943, 2815, 1512, 1455, 1244, 1037 823, 724 (film) | $CDCl_3$ | 1,50-1,80(a.c. 4H); 2,31-2,61 (a.c. 6H); 3,06(m,4H); 3,74 (s,3H); 3,81(t,J=7,0Hz,2H); 6,1(m,2H); 6,6(m,2H); 6,84 (s,4H) |
| 16 | CH | CH | H | CH | MeO- | H | H | Huile | 2940, 2814, 1500, 1451, 1281, 1241, 1028, 743, 723 (film) | $CDCl_3$ | 1,50-1,85(a.c. 4H); 2,33-2,66 (a.c. 6H); 3,10(m,4H); 3,84-3,96(a.c. 5H); 6,12 (t,J=2Hz,2H); 6,65 (t,J=2Hz,2H); 6,93(m,4H) |
| 17 | CH | CH | H | CH | H | H | H | Huile | 2943, 2817, 1600, 1501, 1235, 759, 723, 692 (film) | $CDCl_3$ | 1,41-1,89(a.c. 4H); 2,37 (t,J=7,3Hz,2H); 2,50-2,60 (a.c. 4H); 3,18(m,4H); 3,89 (t,J=6,9Hz,2H); 6,13 (t,J=2,0Hz,2H); 6,64 (t,J=2,0Hz,2H); 6,83-7,33 (a.c. 5H) |
| 18 | N | CH | Cl | CH | H | H | H | 58-61ºC | 2942, 2819, 1600, 1500, 1450, 1381, 1311, 1240, 1140, 966, 756 (KBr) | $CDCl_3$ | 1,47(m,2H); 1,84(m,2H); 2,35 (t,J=7,2Hz,2H); 2,52(m,4H); 3,16(m,4H); 4,04 (t,J=6,8Hz,2H); 6,75-6,94 (a.c. 3H); 7,16(s,1H); 7,23 (s,1H); 7,35(d,J=7,4Hz,2H) |
| 19 | CMe | N | Cl | CCl | H | H | H | Huile | 2944, 2819, 1600, 1532, 1503, 1453, 1404, 1244, 1143, 759, 692 (film) | $CDCl_3$ | 1,43-1,87(a.c. 4H); 2,33 (s,3H); 2,38-2,60(a.c. 6H); 3,17(m,4H); 3,83(t,J=7Hz,2H); 6,9(a.c. 3H); 7,24(m,2H) |

TABLEAU II (suite)

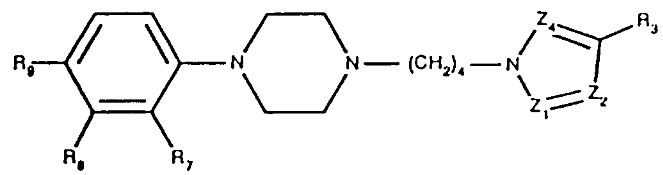

| Exemple | $Z_1$ | $Z_2$ | $R_3$ | $Z_4$ | $R_7$ | $R_8$ | $R_9$ | P.F. | IR $cm^{-1}$ | RMN Solvant | $^1H$-RMN(100 MHz), δ, J=Hz |
|---|---|---|---|---|---|---|---|---|---|---|---|
| 20 | N | CH | Cl | CH | Cl | H | H | Huile | 2943, 2817, 1587, 1480, 1443, 1231, 1040, 971, 751, 612 (film) | DMSO-$d_6$ | 1,40(m,2H); 1,78(m,2H); 2,2-2,6(a.c. 6H); 2,95(m,4H); 4,08(t,J=6,5Hz,2H); 6,95-7,41 (a.c. 4H); 7,50(s,1H); 7,97 (s,1H) |
| 21 | CMe | N | Cl | CCl | Cl | H | H | 89-91ºC | 2936, 2818, 1587, 1531, 1480, 1359, 1243, 1229, 1036, 1016 (KBr) | $CDCl_3$ | 1,3-1,8(a.c. 4H); 2,33(s,3H); 2,35-2,70(a.c. 6H); 2,96 (m,4H); 3,94(t,J=7,2Hz,2H); 6,90-7,50(a.c. 4H) |
| 22 | N | CH | Cl | CH | H | Cl | H | Huile | 2944, 2820, 1594, 1564, 1487, 1451, 1433, 1384, 1239, 987, 980 (film) | $CDCl_3$ | 1,3-1,70(m,2H); 1,70-2,10 (m,2H); 2,39(T,J=7,4Hz,2H); 2,59(m,4H); 3,17(m,4H); 4,09 (t,J=7,4Hz,2H); 6,7-6,9 (a.c. 3H); 7,15(t,J=8.0Hz,1H) 7,37(s,1H); 7,4(s,1H) |

TABLEAU III

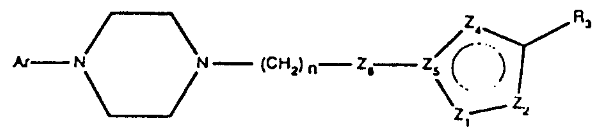

X

| Exemple | $Z_1$ | $Z_2$ | $Z_4$ | $Z_5$ | $Z_6$ | $R_3$ | n | X | Ar | P.F. | IR $cm^{-1}$ | RMN Solvant | $^1$H-RMN(100 MHz),δ,J=Hz |
|---|---|---|---|---|---|---|---|---|---|---|---|---|---|
| 23 | N | CH | CH | N | $CH_2$ | H | 3 | - | 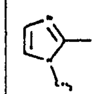 | Huile | 2943, 2812, 1525, 1509, 1469, 1455, 1282, 1137, 751 (film) | $CDCl_3$ | 1,52(m,2H); 1,91(m,2H); 2,30-2,60(a.c. 6H); 3,08 (m,4H); 4,15(t,J=7Hz,2H); 6,22(t,J=2Hz,1H); 6,64 (d,J=1,4Hz,1H); 6,76 (d,J=1,4Hz,1H); 7,38 (d,J=2Hz,1H); 7,48 (d,J=2Hz,1H) |
| 24 | N | CH | CH | N | $CH_2$ | Cl | 1 | - | 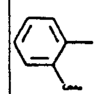 | Huile | 2942, 2819, 1594, 1500, 1450, 1241, 1026, 971, 750 (film) | $CDCl_3$ | 2,70-2,73(a.c. 4H); 2,87 (t,J=6,5Hz,2H); 3,05-3,10 (a.c. 4H); 3,86(s,3H); 4,25 (t,J=6,5Hz,2H); 6,85-7,1 (a.c. 4H); 7,41(s,1H); 7,52 (s,1H) |
| 25 | CMe | N | CCl | N | $CH_2$ | Cl | 3 | citrate | 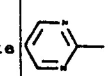 | 137-138°C | 1713, 1618, 1590, 1552, 1511, 1436, 1372, 1247, 1214 (KBr) | DMSO-$d_6$ | 1,51-1,71(a.c. 4H); 2,32 (s,3H); 2,54-2,74(a.c. 10H); 3,72-3,98(a.c. 6H); 6,64 (t,J=4,6Hz,1H); 8,36 (d,J=4,6Hz,2H); 9,75(a.c. 4H) |

TABLEAU III (suite)

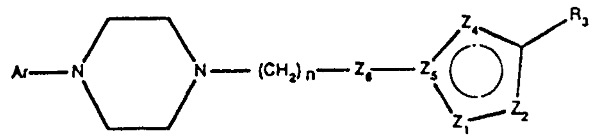

X

| Exemple | $Z_1$ | $Z_2$ | $Z_4$ | $Z_5$ | $Z_6$ | $R_3$ | n | X | Ar | P.F. | IR $cm^{-1}$ | RMN Solvant | $^1H$-RMN (100 MHz), $\delta$, J=Hz |
|---|---|---|---|---|---|---|---|---|---|---|---|---|---|
| 26 | N | CH | CH | N | $CH_2$ | Cl | 3 | - | 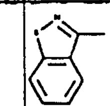 | Huile | 2943, 2815, 1493, 1451, 1423, 1383, 1307, 1261, 970, 739, 613 (film) | $CDCl_3$ | 1,50(m,2H) ; 1,85(m,2H) ; 2,45 (t,J=7,2Hz,2H) ; 2,60 (t,J=4,7Hz,4H) ; 3,53 (t,J=5,0Hz,4H) ; 4,07 (t,J=7,0Hz,2H) ; 7,35(m,4H) ; 7,85(m,2H) |
| 27 | CMe | N | CCl | N | $CH_2$ | Cl | 3 | - | 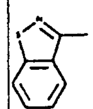 | Huile | 2944, 2816, 1533, 1493, 1422, 1380, 1280, 1246, 1139, 1017, 754, 665 (film) | $CDCl_3$ | 1,55-1,85(a.c. 4H) ; 2,34-2,45 (a.c. 5H) ; 2,62(t,J=4,7Hz,4H) 3,53(t,J=5,0Hz,4H) ; 3,84 (t,J=7,0Hz,2H) ; 7,37(m,2H) ; 7,83(m,2H) |

ACTIVITE BIOLOGIQUE

ACTIVITE ANXIOLITIQUE ET/OU TRANQUILLISANTE

On démontre pour quelques exemples l'activité sur le système nerveux central, et plus précisemment leur activité anxiolytique et tranquillisante, au moyen du test de la réponse d'évitation conditionnée, d'après la méthode de J.S. New et cols (J.S. New, J.P. Yevich, M.S. Eison, D.P. Taylor, A.S. Eison, L.A. Riblet, C.P. Van der Maelen, D.L. Temple, J. Med. Chem. 1986, 29, 1476).

Dans ce test, on utilise des rats Wistar, mâles, de 200 grammes de poids, entraînés pour sauter une barrière dans une cage d'évitation et d'issue (shuttle box) (Letica, référence LI 910 et LI 2700) dans les 30 secondes suivantes à leur introduction dans la cage.

Les produits à activité anxiolytique ou tranquillisante suppriment la réponse d'évitation conditionnée.

Entraînement: premier jour: 11 essais, à intervalles de 3 minutes. Electrochoc sur les pattes, à 30 secondes (5 mA, 0,1 s, 10 s).

Deuxième et troisième jours: 2 essais par jour, uniquement avec les rats sélectionnés [somme des ponctuations du premier jour (excepté le premier essai)>14]

Jour de l'essai: Groupes formés par des rats sélectionnés. Administration orale du produit ou du véhicule 45 minutes avant le début de l'étude.

Dans le Tableau IV on résume les résultats obtenus pour quelques produits.

Compte-tenu de leurs propriétés pharmacodynamiques, des dérivés 1-{4-[4-(2-aryl (ou hétéroaryl)-1-pipérazinyl]-butyl}-1*H*-azoles, selon l'invention, peuvent être utilisés de façon satisfaisante en thérapeutique humaine et animale, en particulier dans le traitement des troubles du système nerveux central, et plus particulièrement pour le traitement de l'anxiété ou comme tranquillisants.

En thérapeutique humaine, la dose d'administration est bien sûr fonction de la gravité de la maladie. Elle sera généralement comprise entre environ 5 et environ 100 mg/jour. Les dérivés de l'invention seront, par exemple, administrés sous forme de comprimés, de solutions ou suspensions, ou bien de gélules.

TABLEAU IV

| INHIBITION DE LA REPONSE D'ELEVATION CONDITIONNEE | | |
|---|---|---|
| Exemple | Activité % (D=80 mg/kg, po) | D.E.$_{50}$ (mg/kg, po) |
| 1 | 8 | - |
| 2 | 38 | - |
| 3 | 82 | 24,5 |
| 4 | 100 | 6,1 |
| 5 | 77 | 15,4 |
| 6 | 100 | 9,3 |
| 7 | 100 | 9,1 |
| 8 | 52 | 61,7 |
| 9 | 88 | 34,5 |
| 10 | 88 | - |
| 11 | 75 | 37,5 |
| 12 | 100 | 8,9 |
| 13 | 95 | 12,8 |
| 14 | 100 | 20,8 |
| 15 | 83 | 50 |
| 16 | 99 | 23,9 |
| 17 | 96 | 23,7 |
| 18 | 100 | 20,7 |
| 19 | 76 | 42 |
| 20 | 86 | 29,5 |
| 21 | 78 | - |
| 22 | 100 | - |
| 23 | 28 | - |
| 24 | 52 | - |
| 25 | 82 | 24,5 |
| 26 | 100 | - |
| 27 | 95 | - |
| Buspirone | 99 | 17,2 |
| Ipsapirone | 98 | 26,1 |

Le produit décrit et les doses testées ont été: 4,5-dichloro-2-méthyl-1-{4-[4-(2-pyrimidinyl)-1-pipérazinyl]-butyl}-1*H*-imidazole citrate.

(Exemple 25): 1 µg/kg p.o. 2 fois par jour.

Les résultats observés ont été consignés dans les tableaux ci-après (V).

Les réponses obtenues avec le produit objet de la présente invention ont été les suivantes:

Le dérivé de l'exemple 42 inhibe le syndrome d'abstinence, qui se manifeste comme une réponse anxiogénique, induit par le diazépam, la cocaïne, l'alcool et la nicotine, et de plus il maintient une réponse anxiolitique significative lorsqu'on arrête l'administration du diazépam, de la cocaïne, de l'alcool ou de la nicotine.

La buspirone maintient le syndrome d'abstinence, qui se manifeste par une réponse anxiogénique, induit par le diazépam et la cocaïne. Cependant, lorsqu'on arrête le traitement avec l'alcool et la nicotine, il ne subsiste plus de syndrome d'abstinence, i.e. d'anxiogénèse. La buspirone inhibe uniquement de façon significative quelques paramètres de réponse anxiogénique après l'arrêt du traitement avec l'alcool.

L'ipsapirone maintient le syndrome d'abstinence, qui se manifeste par une réponse anxiogénique induit par le diazépam, la cocaïne et la nicotine. L'ipsapirone inhibe le syndrome d'abstinence, qui se manifeste sous la forme d'une réponse anxiogénique, induit par l'alcool.

TABLEAU V

EFFET D'UN TRAITEMENT DE COCAINE A LONG TERME ET ARRET, INHIBITION DU SYNDROME D'ABSTINENCE AVEC LE PRODUIT CORRESPONDANT A L'EXEMPLE 25

| TRAITEMENT | REDRESSEMENTS EN 5 MIN. C | O | ACTIVITE EN 5 MIN. C | O | TEMPS EN BOITE O | LAT. C - O |
|---|---|---|---|---|---|---|
| Contrôle | 25 | 87 | 30 | 94 | 60 | 10 |
| Cocaïne | 60 * | 38 * | 68 * | 30 * | 30 * | 18 * |
| Coca. + arrêt 24 h | 7 + | 122 + | 10 + | 149 + | 83 + | 2 + |
| Coca. + arrêt 24 h + Ex. 25 | 86 o * | 20 o * | 92 o * | 23 o * | 30 o * | 26 o * |
| Coca. + arrêt 24 h + Buspirone | 10 + | 98 + | 18 + | 90 + | 70 + | 2 + |
| Coca. + arrêt 24 h + Ipsapirone | 11 + | 89 + | 18 + | 85 + | 62 + | 8 + |

* : p 0.001 (anxiolyse)
\+ : p 0.001 (anxiogénèse)
o : p 0.001 (réversion de l'anxiogénèse)
C - O : Claire - obscure

TABLEAU V (suite)

EFFET D'UN TRAITEMENT DE DIAZEPAM A LONG TERME ET ARRET, INHIBITION DU SYNDROME D'ABSTINENCE AVEC LE PRODUIT CORRESPONDANT A L'EXEMPLE 25

| TRAITEMENT | REDRESSEMENTS EN 5 MIN. C | O | ACTIVITE EN 5 MIN. C | O | TEMPS EN BOITE O | LAT. C - O |
|---|---|---|---|---|---|---|
| Contrôle | 22 | 83 | 36 | 93 | 58 | 12 |
| Diazépam | 70<br>* | 25<br>* | 73<br>* | 24<br>* | 25<br>* | 27<br>* |
| Diaz. + arrêt 24 h | 5<br>+ | 146<br>+ | 5<br>+ | 164<br>+ | 80<br>+ | 1.8<br>+ |
| Diaz. + arrêt 24 h<br>+ Ex. 25 | 85<br>o<br>* | 17<br>o<br>* | 96<br>o<br>* | 19<br>o<br>* | 36<br>o<br>* | 28<br>o<br>* |
| Diaz. + arrêt 24 h<br>+ Buspirone | 9<br>+ | 88<br>+ | 12<br>+ | 100<br>+ | 72<br>+ | 2<br>+ |
| Diaz. + arrêt 24 h<br>+ Ipsapirone | 15<br>+ | 82<br>+ | 17<br>+ | 88<br>+ | 90<br>+ | 8<br>+ |

* : p 0.001 (anxiolyse)
\+ : p 0.001 (anxiogénèse)
o : p 0.001 (réversion de l'anxiogénèse)
C - O : Claire - obscure

TABLEAU V (suite)

EFFET D'UN TRAITEMENT DE ALCOOL A LONG TERME ET ARRET, INHIBITION DU SYNDROME D'ABSTINENCE AVEC LE PRODUIT CORRESPONDANT A L'EXEMPLE 25

| TRAITEMENT | REDRESSEMENTS EN 5 MIN. C | O | ACTIVITE EN 5 MIN. C | O | TEMPS EN BOITE O | LAT. C - O |
|---|---|---|---|---|---|---|
| Contrôle | 22 | 94 | 30 | 102 | 59 | 11 |
| Alcool | 72 * | 22 * | 80 * | 28 * | 28 * | 28 * |
| Alcool + arrêt 24 h | 5 + | 147 + | 6 + | 167 + | 84 + | 2 + |
| Alcool + arrêt 24 h + Ex. 25 | 86 o * | 17 o * | 97 o * | 23 o * | 26 o * | 20 o * |
| Alcool + arrêt 24 h + Buspirone | 18 o | 80 o | 15 | 75 o | 62 | 7 |
| Alcool + arrêt 24 h + Ipsapirone | 21 o | 75 o | 36 o | 80 o | 35 o | 10 o |

* : p 0.001 (anxiolyse)
\+ : p 0.001 (anxiogénèse)
o : p 0.001 (réversion de l'anxiogénèse)
C - O : Claire - obscure

TABLEAU V (suite)

EFFET D'UN TRAITEMENT DE NICOTINE A LONG TERME ET ARRET, INHIBITION DU SYNDROME D'ABSTINENCE AVEC LE PRODUIT CORRESPONDANT A L'EXEMPLE 25

| TRAITEMENT | REDRESSEMENTS EN 5 MIN. C | O | ACTIVITE EN 5 MIN. C | O | TEMPS EN BOITE O | LAT. C - O |
|---|---|---|---|---|---|---|
| Contrôle | 22 | 83 | 36 | 93 | 58 | 12 |
| Nicotine | 66 * | 23 * | 70 * | 25 * | 25 * | 26 * |
| Nic. + arrêt 24 h | 10 + | 125 + | 14 + | 147 + | 78 + | 2 + |
| Nic. + arrêt 24 h + Ex. 25 | 90 o * | 18 o * | 102 o * | 20 o * | 29 o * | 23 o * |
| Nic. + arrêt 24 h + Buspirone | 12 | 80 | 20 | 85 | 65 | 8 |
| Nic. + arrêt 24 h + Ipsapirone | 9 o | 84 o | 18 o | 98 o | 70 o | 3 o |

* : p 0.001 (anxiolyse)
\+ : p 0.001 (anxiogénèse)
o : p 0.001 (réversion de l'anxiogénèse)
C - O : Claire - obscure

Les dérivés de formule générale I selon l'invention sont donc utiles comme substances actives de médicaments destinés au traitement de troubles associés au syndrome d'abstinence qui se manifeste en particulier sous la forme d'une réponse anxiogénique, induit par la suppression brusque d'un traitement prolongé avec des benzodiazépines telles que le diazépam, la cocaïne l'alcool et/ou la nicotine.

En thérapeutique humaine, la dose d'administration est bien sûr fonction de la gravité du syndrome.

Elle sera généralement comprise entre environ 5 et environ 100 mg/jour.

ACTIVITE ANTIHYPERTENSIVE

On démontre pour quelques exemples l'activité sur le système cardiovasculaire en particulier leur activité antihypertensive. Cette activité est manifestée par la protection des animaux devant la mort induite par une injection intraveineuse de norepinefrine, laquelle produit une crise hypertensive aux rats.

Test de l'antagonisme de la norepinefrine chez le rat

On démontre l'activité antihypertensive au moyen du test décrit par P.A.J. Janssen et cols. (P.A.J. Janssen, C.J.E. Niemegeers, K.H.L. Schellekens, F.J. Verbruggen et J.M. Van Nueten, Arzneim. Forsch., 1963, 13, 205).

Dans ce test, on utitise des rats Wistar, mâles, de 200 grammes de poids. On administre le produit à étudier par voie intrapéritonéale et après 2 heures on administre 1,25 mg/kg (i.v.) de norepinefrine. Ce traitement provoque la mort des animaux contrôle.

La dose initiale d'administration dans ces études est 40 mg/kg (i.p.).

On détermine la DE-50 des produits les plus actifs et on résume les résultats obtenus dans le tableau VI.

TABLEAU VI

| ACTIVITE ANTIHYPERTENSIVE ANTAGONISTE DE LA NOREPINEFRINE | | |
|---|---|---|
| Exemple | Activité % (D=40 mg/kg, po) | $D.E._{50}$ (mg/kg, po) |
| 8 | 100 | - |
| 11 | 100 | 14 |
| 12 | 100 | 0,46 |
| 13 | 100 | 0,18 |
| 14 | 100 | 8,9 |
| 16 | 100 | 2,23 |
| 17 | 100 | 10 |
| 18 | 100 | 4,1 |
| 19 | 100 | 7,6 |
| 20 | 100 | 1,9 |
| 21 | 100 | 1,3 |
| 24 | 100 | 5 |
| 26 | 66 | - |
| 27 | 100 | - |
| Clonidine | 100 | 1,58 |
| Tolazoline | 100 | 2,97 |

ACTIVITE SUR L'AMELIORATION DE LA COGNITION

On démontre pour l'exemple 25 l'activité sur l'amélioration de la cognition :

On étudie l'influence de ce produit sur le processus d'accoutumance des souris dans le test de la boite claire/obscure décrit par J.M. Barnes et cols. (Pharmacol. Biochem. Behav., 1990, 35, 955-962). On étudie d'un côté l'effet sur l'apprentissage (la vitesse d'accoutumance) et d'un autre côté la capacité de blocage des effets contraires produits par l'escopolamine.

On place la souris dans la zone claire d'une boîte divisée en deux compartiments, un très illuminé, boite claire, et l'autre peu illuminé, boîte obscure.

1) On compte le nombre de fois que la souris se dresse sur les pattes arrières dans chaque compartiment pendant 5 minutes. (voir colonne 1 du tableau ci-après)
2) L'activité dans chaque compartiment est donnée par le comptage du nombre de croisements par les carreaux qui constituent les divisions de chaque compartiment. (voir colonne 2 du tableau ci-après)
3) On mesure le temps passé dans la boîte obscure pendant les 5 minutes du comptage. (voir colonne 3 du tableau ci-après)
4) On détermine la latence initiale, c'est-à-dire le temps passé depuis que l'on place l'animal dans la boîte claire, au début de l'essai, jusqu'à son entrée dans la boîte obscure. (voir colonne 4 du tableau ci-après

On administré aux animaux témoins deux traitements de véhicule par jour. On administre aux animaux traités avec le composé 29 deux doses par jour de 0.00001 ng/kg, po. de ce produit. La même opération est répétée journellement pendant 5 jours.

Les animaux apprennent à rester plus de temps dans la boîte obscure et à s'y rendre plus rapidement.

Au sixième jour on administre de l'escopolamine (2 x 0,25 mg/kg, ip). Avec ce traitement les animaux du groupe témoin "oublient" le comportement appris consistant à rester plus de temps dans la boîte obscure.

Au septième jour ils recupèrent l'habitude apprise.

Le traitement avec un produit qui améliore la cognition fait que:

1) Le comportement appris est amélioré, l'apprentissage est fait plus rapidement et le temps de résidence dans la boîte obscure augmente.
2) La réversion de l'apprentissage produite par l'escopolamine est totallement bloquée.

Les données obtenues sont résumées dans le tableau VII. Elles démontrent que le composé 25 a une activité d'amélioration de la cognition, étant donné que ce composé améliore le processus de l'apprentissage et bloque les effets de l'escopolamine.

Le piracetam essayé sous les mêmes conditions n'a aucune activité.

TABLEAU VII

INFLUENCE DE L'EXEMPLE 25 (0.00001 ng/Kg, PO, DEUX FOIS PAR JOUR) SUR LE PROCESSUS D'ACCOUTUMANCE CHEZ LA SOURIS DETERMINE PAR LE TEST DE LA BOITE CLAIRE/OBSCURE.

| JOUR DE TRAITEMENT | REDRESSEMENTS EN 5 MIN. TEMOIN C | TEMOIN O | EXEMPLE C | EXEMPLE O | ACTIVITE EN 5 MIN TEMOIN C | TEMOIN O | EXEMPLE C | EXEMPLE O | TEMPS EN BOITE TEMOIN O | EXEMPLE O | LATENCE TEMOIN C - O | EXEMPLE C - O |
|---|---|---|---|---|---|---|---|---|---|---|---|---|
| 1 | 22 | 72 | 23 | 79 | 34 | 79 | 36 | 82 | 60 | 60 | 11 | 11 |
| 2 | 26 | 76 | 10 * | 147 * | 36 | 83 | 11 * | 167 * | 61 | 80 * | 12 | 2 * |
| 3 | 24 | 74 | 8 * | 156 * | 34 | 78 | 10 * | 179 * | 60 | 79 * | 11 | 2 * |
| 4 | 9 * | 147 * | 9 * | 161 * | 12 * | 167 * | 9 * | 183 * | 80 * | 83 * | 2 * | 1.5 * |
| 5 | 9 * | 138 * | 11 * | 151 * | 12 * | 171 * | 12 * | 176 * | 79 * | 82 * | 2.5 * | 1 * |
| 6 (*) | 59 + | 38 + | 10 * o | 172 * o | 67 + | 30 + | 14 * o | 181 * o | 27 + | 81 * o | 21 + | 1.5 * o |
| 7 | 8 * | 154 * | 8 * | 181 * | 13 * | 163 * | 8 * | 187 * | 81 * | 83 * | 2.5 * | 1 * |

(*) 2 traitements préalables avec de l'escopolamine (0.25 mg/kg, ip)
* $p < 0.001$ (amélioration de l'apprentissage, comparé avec le jour 1)
\+ $p < 0.001$ (réversion du procès d'accoutumance produit par l'escopolamine)
o $p < 0.001$ (inhibition de l'effet de l'escopolamine)
C - O : Claire - obscure

ACTIVITE ANTIDEPRESSIVE

On démontre l'activité antidépressive pour l'exemple 25. On utilise le test de comportement désespéré chez la souris decrit par R.D. Porsolt et cols. (Arch. Int. Pharmacodyn.. 1977. 229. 327-336).

Les animaux sont placés, pendant 6 minutes, dans un cylindre contenant de l'eau, dont ils ne peuvent pas s'échapper. On mesure, en groupes de 10 souris par dose testée, la durée de l'immobilité entre les minutes 2ème et 5ème.

Le produit étudié est administré par voie i.p. 1 heure avant le test. Dans ce test on explique l'immobilité des animaux comme produite par leur état dépressif conséquence d'être mis face à une situation adverse et insoluble dans un environnement hostile comme l'eau.

Les antidépressifs réduisent cette immobilité.

Dans notre essai on a utilisé l'imipramine (30 mg/kg.ip) comme produit de référence.

Les résultats démontrent que le composé 25 a une activité antidépressive puis qu'il réduit significativement le temps d'immobilité du groupe témoin.

| Produit | Doses (mg/kg, ip) | Temps d'immobilité (secondes) |
|---|---|---|
| Témoin | - | 118 |
| Exemple 25 | 1 | 64 ($p < 0.05$) |
| Imipramine | 30 | 71 ($p < 0.05$) |

On indiquera ci-après, à titre d'exemples, deux formes galéniques particulières des dérivés objet de la présente invention.

| Exemple de formule par comprimé | |
|---|---|
| Composé 25 | 5 mg |
| Lactose | 60 mg |
| Cellulose microcristalline | 25 mg |
| Povidone | 5 mg |
| Amidon prégélatinisé | 3 mg |
| Dioxyde de silice colloïdale | 1 mg |
| Stearate de magnésium | 1 mg |
| Poids comprimé | 100 mg |

| Exemple de formule par gélule | |
|---|---|
| Composé 25 | 10 mg |
| Glycéride polyoxyéthylenée | 135 mg |
| Béhénate de glycérine | 5 mg |
| excipient: gélatine molle q.s. | 150 mg |

## Revendications

1. Composés hétérocycliques caractérisés en ce qu'ils répondent à la formule générale I

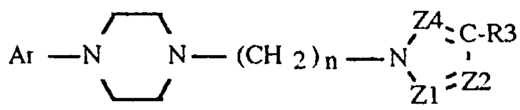

dans laquelle

Ar représente un radical aromatique azoté ou non, choisi parmi les groupes phényle, phényle substitué par un radical méthoxy, 2-pyrimidine, 2-N-méthylimidazol et 3-(1,2-benzisothiazole),

n peut prendre les valeurs 2 à 7,

$Z_1$ représente un atome d'azote ou un groupement C-$R_1$,
$Z_2$ représente un atome d'azote ou un groupement C-$R_2$,
$Z_4$ représente un atome d'azote ou un groupement C-$R_4$,

et
$R_1$, $R_2$, $R_3$ et $R_4$, identiques ou différents pouvant également former une partie d'un autre cycle, aromatique ou non, représentent un atome d'hydrogène, un halogène, un radical alkyle inférieur, un radical carboxamido, un radical alkyloxy-carbonyle, ou un radical phényle,

- à la condition que lorsque Ar représente le groupe 2-pyrimidine, le radical hétéroaromatique

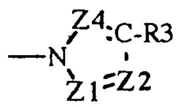

est choisi parmi le groupe comprenant les radicaux imidazolyle trisubstitués pour lesquels

Z1 représente un groupement C-R1,
Z2 représente un atome d'azote,
Z4 représente un groupement C-R4, et

R1, R3 et R4, identiques ou différents, sont différents d'un atome d'hydrogène, et les radicaux benzimidazolyle substitués ou non, pour lesquels

Z1 représente un groupement C-R1,
Z2 représente un atome d'azote,
Z4 représente un groupement C-R4, et

R3 et R4 représentent un radical -CH=CH-CH=CH-et leurs sels d'addition des acides pharmaceutiquement acceptables,

- à l'exception des composés pour lesquels Ar représente un groupe phényle, substitué ou non, $Z_1$ représente un groupement C-$R_1$, $Z_2$ représente un atome d'azote,$Z_4$ représente un groupement C-$R_4$, et $R_3$-$R_4$ forment une partie d'un autre cycle aromatique, et

- à l'exception des composés pour lesquels Ar représente un groupe phényle, substitué ou non, et le radical hétéroaromatique

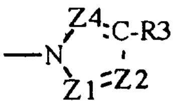

représente un groupe 4, 5, 6 ou 7-azaindolyle.

2. Les composés répondant à la formule générale I selon la revendication 1, séléctionnés parmi le groupe suivant:

- 2,4,5,-triphényl-1-{4-[4-(2-pyrimidinyl)-1-pipérazinyl]-butyl}-1*H*-imidazole.
- 4,5-diphényl-2-méthyl-1-{4-[4-(2-pyrimidinyl)-1-pipérazinyl]-butyl}-1*H*-imidazole.
- 4,5,-dichloro-2-methyl-1-{4-[4-(2-pyrimidinyl)-1-pipérazinyl]-butyl}-1*H*-imidazole.
- 1-{4-[4-(2-pyrimidinyl)-1-pipérazinyl]-butyl}-1*H*-benzimidazole,
- 2-chloro-1-{4-[4-(2-pyrimidinyl)-1-pipérazinyl]-butyl}-1*H*-benzimidazole.
- 5-chloro-1-{4-[4-(2-pyrimidinyl)-1-pipérazinyl]-butyl}-1*H*-benzimidazole.
- 6-chloro-1-{4-[4-(2-pyrimidinyl)-1-pipérazinyl]-butyl}-1*H*-benzimidazole.
- 2-méthyl-1-{4-[4-(2-pyrimidinyl)-1-pipérazinyl]-butyl}-1*H*-benzimidazole.
- 5,6-diméthyl-1-{4-[4-(2-pyrimidinyl)-1-pipérazinyl]-butyl}-1*H*-benzimidazole.
- 4-chloro-1-{4-[4-(4-méthoxyphényl)-1-pipérazinyl]-butyl}-1*H*-pyrazole
- 4,5-dichloro-2-méthyl-1-{4-[4-(4-méthoxyphényl)-1-pipérazinyl]-butyl}-1*H*-imidazole,
- 4-chloro-1-{4-[4-(2-méthoxyphényl)-1-pipérazinyl]-butyl}-1*H*-pyrazole,
- 4,5-dichloro-2-méthyl-1-{4-[4-(2-méthoxyphényl)-1-pipérazinyl]-butyl}-1*H*-imidazole,
- 4-chloro-1-{4-[4-(3-méthoxyphényl)-1-pipérazinyl]-butyl}-1*H*-pyrazole,
- 1-{4-[4-(4-méthoxyphényl)-1-pipérazinyl]-butyl}-pyrrole,
- 1-{4-[4-(2-méthoxyphényl)-1-pipérazinyl]-butyl}-pyrrole,
- 1-{4-[4-(phényl)-1-pipérazinyl]-butyl}-pyrrole,
- 4-chloro-1-{4-[4-(phényl)-1-pipérazinyl]-butyl}-1*H*-pyrazole,
- 4,5-dichloro-2-méthyl-1-{4-[4-(phényl)-1-pipérazinyl]-butyl}-1*H*-imidazole,
- 4-chloro-1-{4-[4-(2-chlorophényl)-1-pipérazinyl]-butyl}-1*H*-pyrazole,
- 4,5-dichloro-2-méthyl-1-{4-[4-(2-chlorophényl)-1-pipérazinyl]-butyl}-1*H*-imidazole,
- 4-chloro-1-{4-[4-(3-chorophényl)-1-pipérazinyl]-butyl}-1*H*-pyrazole,
- 1-{4-[4-(2-N-méthylimidazolyl)-1-pipérazinyl]-butyl}-1*H*-pyrazole,
- 4-chloro-1-{2-[4-(2-méthoxyphényl)-1-pipérazinyl]-éthyl}-1*H*-pyrazole,
- 4,5-dichloro-2-méthyl-1-{4-[4-(2-pyrimidinyl)-1-pipérazinyl]-butyl}-1*H*-imidazole citrate salt,
- 4-chloro-1-{4-[4-(3-(1,2-benzisothiazole))-1-pipérazinyl]-butyl}-1*H*-pyrazole,
- 4,5-dichloro-2-méthyl-1-{4-[4-(3-(1,2-benzisothiazole))-1-pipérazinyl] -butyl}-1*H*-imidazole,

3. Procédé de préparation des composés selon l'une des revendications 1 et 2, caracterisé par la mise en oeuvre d'une opération de réaction d'un composé de formule générale II

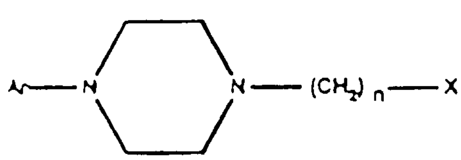

(II)

dans laquelle

Ar et n ont les significations mentionnées selon la revendication 1 et X représente un atome d'halogène, ou

un groupe partant choisi parmi le tosyloxy ou le mésiloxy, avec un composé de formule générale III

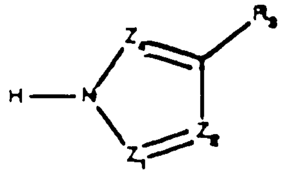

(III)

dans laquelle

$Z_1$, $Z_2$, $Z_4$, et $R_3$ ont les significations mentionnées dans la revendication 1

**4.** Procédé de préparation des composés selon l'une des revendications 1 et 2, caracterisé par la mise en oeuvre d'une opération de réaction d'un composé de formule générale IV

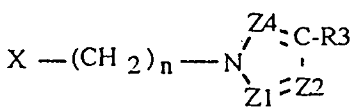

(IV)

dans laquelle

$Z_1$, $Z_2$, $Z_4$, $R_3$, n et X ont les mêmes significations mentionnées dans les revendications 1 et 3, avec un composé de formule générale V

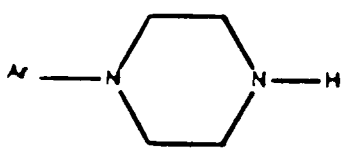

(V)

dans laquelle

Ar a les significations mentionnées dans la revendication 1.

5. Procédé de préparation des composés selon l'une des revendications 1 et 2, caractérisé par la mise en oeuvre d'une opération de réaction d'un composé de formule générale VI

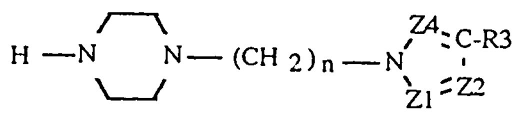

(VI)

dans laquelle

$Z_1$, $Z_2$, $Z_4$, $R_3$, et n ont les significations mentionnées dans la revendication 1, avec un composé de formule générale VII.

Ar—X (VII)

dans laquelle

Ar et X ont les mêmes slgnifications mentionnées dans la revendication 3.

6. Procédé de préparation des composés selon l'une des revendications 1 et 2, caractérisé par la mise en oeuvre d'une opération de réaction d'un composé de formule générale VIII

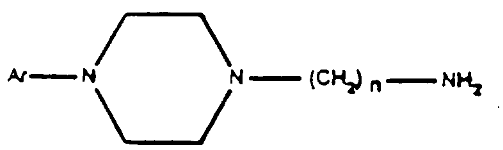

(VIII)

dans laquelle

Ar et n ont les significations mentionnées dans la revendication 1, avec le 2,5-diméthoxytétrahydrofurane.

7. Procédé de préparation des composés selon l'une des revendications 1 et 2, caractérisé par la mise en oeuvre d'une opération de réaction d'un composé de formule générale I dans laquelle Ar, n, $Z_1$, $Z_2$, $Z_4$, et $R_3$ ont les significations mentionnées dans la revendication 1 mais où l'un au moins des substituants $R_1$, $R_2$, $R_3$ et $R_4$ représente un radical carboxylique, avec une amine.

8. Procédé de préparation des composés selon l'une des revendications 1 et 2, caractérisé par la mise en oeuvre d'une opération de réduction d'un composé de formule générale IX

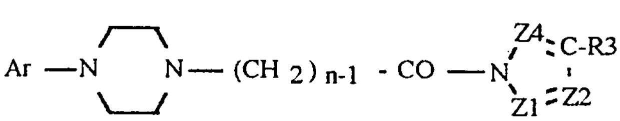

(IX)

dans laquelle Ar, Z1, Z2, Z4, R3 et n ont les significations mentionnées selon la revendication 1.

9. A titre de médicaments, les dérivés selon l'une des revendications 1 ou 2, et leurs sels physiologiquement acceptables.

**10.** Utilisation des dérivés selon l'une des revendications 1 ou 2, et leurs sels physiologiquement acceptables, pour la fabrication d'un médicament destinné au traitement de l'anxiété, en particulier pour la fabrication de tranquillisants et/ou d'anxiolytiques, de la dépression, du syndrome d'abstinence, des troubles de la cognition et de l'hypertension.

**11.** Compositions pharmaceutiques, caractérisées en ce qu'elles contiennent, outre un support pharmaceutiquement acceptable, au moins un dérivé selon l'une des revendications 1 ou 2, ou l'un de ses sels pharmaceutiquement acceptables.

## Claims

**1.** Heterocyclic compounds, characterized in that they have the general formula I

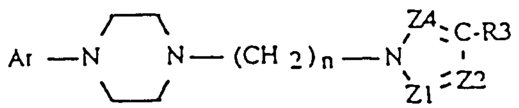

(I)

in which

Ar represents an aromatic radical which may or may not contain nitrogen and which is chosen from phenyl groups, phenyl substituted by a methoxy radical, 2-pyrimidine, 2-N-methylimidazole and 3-(1,2-benzisothiazole),

n may take the values 2 to 7,

$Z_1$ represents a nitrogen atom or a C-$R_1$ group,
$Z_2$ represents a nitrogen atom or a C-$R_2$ group,
$Z_4$ represents a nitrogen atom or a C-$R_4$ group,

and
$R_1$, $R_2$, $R_3$ and $R_4$, which are identical or different and may also form part of another aromatic or nonaromatic ring, represent a hydrogen atom, a halogen, a lower alkyl radical, a carboxamido radical, an alkyloxycarbonyl radical or a phenyl radical,

- on the condition that when Ar represents the 2-pyrimidine group the heteroaromatic radical

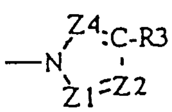

is chosen from the group including the trisubstituted imidazolyl radicals in which

Z1 represents a C-R1 group,
Z2 represents a nitrogen atom,
Z4 represents a C-R4 group, and

R1, R3 and R4, which are identical or different, are other than a hydrogen atom,
and the substituted or unsubstituted benzimidazolyl radicals in which

Z1 represents a C-R1 group,
Z2 represents a nitrogen atom,
Z4 represents a C-R4 group, and

R3 and R4 represent a radical -CH=CH-CH=CH-and their addition salts of pharmaceutically acceptable acids,

- with the exception of the compounds in which Ar represents a phenyl group, substituted or otherwise, $Z_1$ represents a C-$R_1$ group, $Z_2$ represents a nitrogen atom, $Z_4$ represents a C-$R_4$ group and $R_3$-$R_4$ form a part of another aromatic ring, and
- with the exception of the compounds in which Ar represents a phenyl group, substituted or otherwise, and the heteroaromatic radical

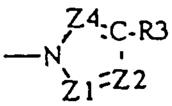

represents a 4, 5, 6 or 7-azaindolyl group.

**2.** The compounds corresponding to the general formula I according to Claim 1, selected from the following group:

- 2,4,5-triphenyl-1-{4-[4-(2-pyrimidinyl)-1-piperazinyl]-butyl}-1H-imidazole,
- 4,5-diphenyl-2-methyl-1-{4-[4-(2-pyrimidinyl)-1-piperazinyl]-butyl}-1H-imidazole,
- 4,5-dichloro-2-methyl-1-{4-[4-(2-pyrimidinyl)-1-piperazinyl]-butyl}-1H-imidazole,
- 1-{4-[4-(2-pyrimidinyl)-1-piperazinyl]-butyl}-1H-benzimidazole,
- 2-chloro-1-{4-[4-(2-pyrimidinyl)-1-piperazinyl]-butyl}-1H-benzimidazole,
- 5-chloro-1-{4-[4-(2-pyrimidinyl)-1-piperazinyl]-butyl}-1H-benzimidazole,
- 6-chloro-1-{4-[4-(2-pyrimidinyl)-1-piperazinyl]-butyl}-1H-benzimidazole,
- 2-methyl-1-{4-[4-(2-pyrimidinyl)-1-piperazinyl]-butyl}-1H-benzimidazole,
- 5,6-dimethyl-1-{4-[4-(2-pyrimidinyl)-1-piperazinyl]-butyl}-1H-benzimidazole,
- 4-chloro-1-{4-[4-(4-methoxyphenyl)-1-piperazinyl]-butyl}-1H-pyrazole
- 4,5-dichloro-2-methyl-1-{4-[4-(4-methoxyphenyl)-1-piperazinyl]-butyl}-1H-imidazole,
- 4-chloro-1-{4-[4-(2-methoxyphenyl)-1-piperazinyl]-butyl}-1H-pyrazole,
- 4,5-dichloro-2-methyl-1-{4-[4-(2-methoxyphenyl)-1-piperazinyl]-butyl}-1H-imidazole,
- 4-chloro-1-{4-[4-(3-methoxyphenyl)-1-piperazinyl]-butyl}-1H-pyrazole,
- 1-{4-[4-(4-methoxyphenyl)-1-piperazinyl]-butyl}-pyrrole,
- 1-{4-[4-(2-methoxyphenyl)-1-piperazinyl]-butyl}-pyrrole,
- 1-{4-[4-phenyl-1-piperazinyl]-butyl}-pyrrole,
- 4-chloro-1-{4-[4-phenyl-1-piperazinyl]-butyl}-1H-pyrazole,
- 4,5-dichloro-2-methyl-1-{4-[4-phenyl-1-piperazinyl]-butyl}-1H-imidazole,
- 4-chloro-1-{4-[4-(2-chlorophenyl)-1-piperazinyl]-butyl}-1H-pyrazole,
- 4,5-dichloro-2-methyl-1-{4-[4-(2-chlorophenyl)-1-piperazinyl]-butyl}-1H-imidazole,
- 4-chloro-1-{4-[4-(3-chlorophenyl)-1-piperazinyl]-butyl}-1H-pyrazole,
- 1-{4-[4-(2-N-methylimidazolyl)-1-piperazinyl]-butyl}-1H-pyrazole,
- 4-chloro-1-{2-[4-(2-methoxyphenyl)-1-piperazinyl]-ethyl}-1H-pyrazole,
- 4,5-dichloro-2-methyl-1-{4-[4-(2-pyrimidinyl)-1-piperazinyl]-butyl}-1H-imidazole citrate salt,
- 4-chloro-1-{4-[4-(3-(1,2-benzisothiazolyl))-1-piperazinyl]-butyl}-1H-pyrazole and
- 4,5-dichloro-2-methyl-1-{4-[4-(3-(1,2-benzisothiazolyl))-1-piperazinyl]-butyl}-1H-imidazole.

**3.** Process for the preparation of the compounds according to one of Claims 1 and 2, characterized by the use of an operation involving reaction of a compound of general formula II

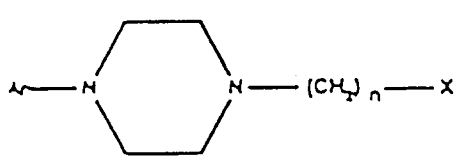

(II)

in which

Ar and n have the meanings mentioned according to Claim 1 and X represents a halogen atom or a leaving

group chosen from tosyloxy or mesyloxy, with a compound of general formula III

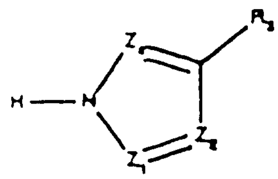

(III)

in which

$Z_1$, $Z_2$, $Z_4$ and $R_3$ have the meanings mentioned in Claim 1.

**4.** Process for the preparation of the compounds according to one of Claims 1 and 2, characterized by the use of an operation involving reaction of a compound of general formula IV

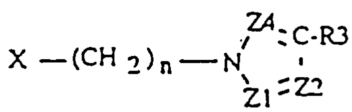

IV

in which

$Z_1$, $Z_2$, $Z_4$, $R_3$, n and X have the same meanings mentioned in Claims 1 and 3,

with a compound of general formula V

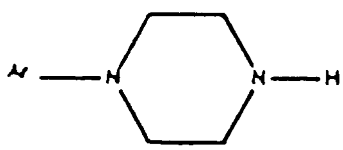

(V)

in which

Ar has the meanings mentioned in Claim 1.

**5.** Process for the preparation of the compounds according to one of Claims 1 and 2, characterized by the use of an operation involving reaction of a compound of general formula VI

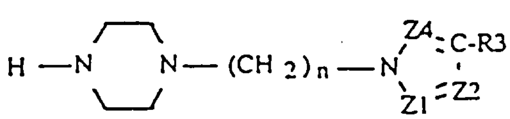

(VI)

in which

$Z_1$, $Z_2$, $Z_4$, $R_3$ and n have the meanings mentioned in Claim 1, with a compound of general formula VII

$$Ar—X \qquad (VII)$$

in which

Ar and X have the same meanings mentioned in Claim 3.

6. Process for the preparation of the compounds according to one of Claims 1 and 2, characterized by the use of an operation involving reaction of a compound of general formula VIII

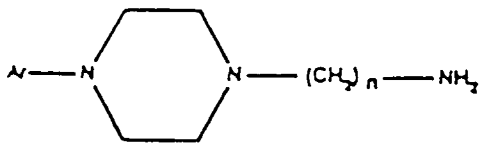

(VIII)

in which

Ar and n have the meanings mentioned in Claim 1, with 2,5-dimethoxytetrahydrofuran.

7. Process for the preparation of the compounds according to one of Claims 1 and 2, characterized by the use of an operation involving reaction of a compound of general formula I in which Ar, n, $Z_1$, $Z_2$, $Z_4$ and $R_3$ have the meanings mentioned in Claim 1, but where at least one of the substituents $R_1$, $R_2$, $R_3$ and $R_4$ represents a carboxyl radical, with an amine.

8. Process for the preparation of the compounds according to one of Claims 1 and 2, characterized by the use of an operation involving reduction of a compound of general formula IX

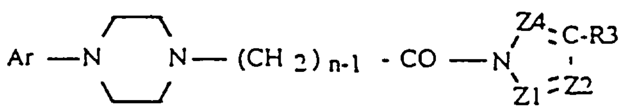

(IX)

in which

Ar, $Z_1$, $Z_2$, $Z_4$, $R_3$ and n have the meanings mentioned according to Claim 1.

9. As medications, the derivatives according to one of Claims 1 and 2, and their physiologically acceptable salts.

10. Use of the derivatives according to one of Claims 1 and 2, and their physiologically acceptable salts, for the production of a medication intended for the treatment of anxiety, in particular for the production of tranquillizers and/or anxiolytic agents, and of medications intended for the treatment of depression, the abstinence syndrome, cognition disorders and hypertension.

11. Pharmaceutical compositions, characterized in that they contain, in addition to a pharmaceutically acceptable excipient, at least one derivative according to one of Claims 1 or 2, or one of its physiologically acceptable salts.

**Patentansprüche**

1. Heterocyclische Verbindungen, dadurch gekennzeichnet, daß sie die allgemeine Formel (I) haben:

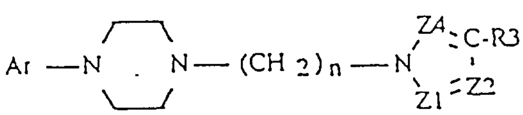

(I)

in der bedeuten:

| | |
|---|---|
| Ar | einen aromatischen Rest, der Stickstoff enthält oder nicht enthält, ausgewählt aus den Phenyl-, durch einen Methoxy-Rest substituierten Phenyl-, 2-Pyrimidin-, 2-N-Methylimidazol- und 3-(1,2-Benzisothiazol)-Gruppen, |
| n | eine Zahl von 2 bis 7, |
| $Z_1$ | ein Stickstoffatom oder eine C-$R_1$-Gruppe, |

$Z_2$ ein Stickstoffatom oder eine $C-R_2$-Gruppe,
$Z_4$ ein Stickstoffatom oder eine $C-R_4$-Gruppe und
$R_1$, $R_2$, $R_3$ und $R_4$, die identisch oder verschieden sind und die auch einen Teil eines anderen aromatischen oder nicht-aromatischen Ringes bilden können, ein Wasserstoffatom, ein Halogenatom, einen niederen Alkylrest, einen Carboxamidorest, einen Alkyloxycarbonylrest oder einen Phenylrest,

- mit der Maßgabe, daß dann, wenn Ar für die 2-Pyrimidin-Gruppe steht, der heteroaromatische Rest

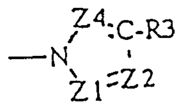

ausgewählt wird aus der Gruppe, die umfaßt

die trisubstituierten Imidazolyl-Reste, in denen

$Z_1$ steht für eine $C-R_1$-Gruppe,
$Z_2$ steht für ein Stickstoffatom
$Z_4$ steht für eine $C-R_4$-Gruppe und
$R_1$, $R_3$ und $R_4$, die identisch oder verschieden sind, von einem Wasserstoffatom verschieden sind, und

die substituierten oder unsubstituierten Benzimidazolyl-Reste, in denen

$Z_1$ steht für eine $C-R_1$-Gruppe,
$Z_2$ steht für ein Stickstoffatom
$Z_4$ steht für eine $C-R_4$-Gruppe und
$R_3$ und $R_4$ stehen für einen CH=CH-CH=CH-Rest

und ihre pharmazeutisch akzeptablen Säureadditionssalze,

- ausgenommen die Verbindungen, in denen stehen Ar für eine substituierte oder unsubstituierte Phenylgruppe, $Z_1$ für eine $C-R_1$-Gruppe, $Z_2$ ein Stickstoffatom, $Z_4$ für eine $C-R_4$-Gruppe und $R_3$ und $R_4$ einen Teil eines anderen aromatischen Ringes bilden, und
- ausgenommen die Verbindungen, in denen stehen Ar für eine substituierte oder unsubstituierte Phenylgruppe und der heteroaromatische Rest

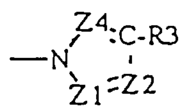

für eine 4,5,6 oder 7-Azaindolyl-Gruppe.

**2.** Verbindungen der allgemeinen Formel (I) nach Anspruch 1, die ausgewählt werden aus der folgenden Gruppe

- 2,4,5-Triphenyl-1-{4-[4-(2-pyrimidinyl)-1-piperazinyl]-butyl}-1H-imidazol,
- 4,5-Diphenyl-2-methyl-1-{4-[4-(2-pyrimidinyl)-1-piperazinyl]-butyl}-1H-imidazol,
- 4,5-Dichloro-2-methyl-1-{4-[4-(2-pyrimidinyl)-1-piperazinyl]-butyl}-1H-imidazol,
- 1-{4-[4-(2-Pyrimidinyl)-1-piperazinyl]-butyl}-1H-benzimidazol,
- 2-Chloro-1-{4-[4-(2-pyrimidinyl)-1-piperazinyl]-butyl}-1H-benzimidazol,
- 5-Chloro-1-{4-[4-(2-pyrimidinyl)-1-piperazinyl]-butyl}-1H-benzimidazol,
- 6-Chloro-1-{4-[4-(2-pyrimidinyl)-1-piperazinyl]-butyl}-1H-benzimidazol,
- 2-Methyl-1-{4-[4-(2-pyrimidinyl)-1-piperazinyl]-butyl}-1H-benzimidazol,

- 5,6-Dimethyl-1-{4-[4-(2-pyrimidinyl)-1-piperazinyl]-butyl}-1H-benzimidazol,
- 4-Chloro-1-{4-[4-(4-methoxyphenyl)-1-piperazinyl]-butyl}-1H-pyrazol,
- 4,5-Dichloro-2-methyl-1-{4-[4-(4-methoxyphenyl)-1-piperazinyl]-butyl}-1H-imidazol,
- 4-Chloro-1-{4-[4-(2-methoxyphenyl)-1-piperazinyl]-butyl}-1H-pyrazol,
- 4,5-Dichloro-2-methyl-1-{4-[4-(2-methoxyphenyl)-1-piperazinyl]-butyl}-1H-imidazol,
- 4-Chloro-1-{4-[4-(3-methoxyphenyl)-1-piperazinyl]-butyl}-1H-pyrazol,
- 1-{4-[4-(4-Methoxyphenyl)-1-piperazinyl]-butyl}-pyrrol,
- 1-{4-[4-(2-Methoxyphenyl)-1-piperazinyl]-butyl}-pyrrol,
- 1-{4-[4-(Phenyl)-1-piperazinyl]-butyl}-pyrrol,
- 4-Chloro-1-{4-[4-(phenyl)-1-piperazinyl]-butyl}-1H-pyrazol,
- 4,5-Dichloro-2-methyl-1-{4-[4-(phenyl)-1-piperazinyl]-butyl}-1H-imidazol,
- 4-Chloro-1-{4-[4-(2-chlorophenyl)-1-piperazinyl]-butyl}-1H-pyrazol,
- 4,5-Dichloro-2-methyl-1-{4-[4-(2-chlorophenyl)-1-piperazinyl]-butyl}-1H-imidazol,
- 4-Chloro-1-{4-[4-(3-chlorophenyl)-1-piperazinyl]-butyl}-1H-pyrazol,
- 1-{4-[4-(2-N-Methylimidazolyl)-1-piperazinyl]-butyl}-1H-pyrazol,
- 4-Chloro-1-{2-[4-(2-methoxyphenyl)-1-piperazinyl]-ethyl}-1H-pyrazol,
- 4,5-Dichloro-2-methyl-1-{4-[4-(2-pyrimidinyl)-1-piperazinyl]-butyl}-1H-imidazol-citratsalz,
- 4-Chloro-1-{4-[4-(3(1,2-benzisothiazol))-1-piperazinyl]-butyl}-1H-pyrazol,
- 4,5-Dichloro-2-methyl-1-{4-[4-(3(1,2-benzisothiazol))-1-piperazinyl]-butyl}-1H-imidazol.

**3.** Verfahren zur Herstellung der Verbindungen nach einem der Ansprüche 1 und 2, dadurch gekennzeichnet, daß man eine Verbindung der allgemeinen Formel (II)

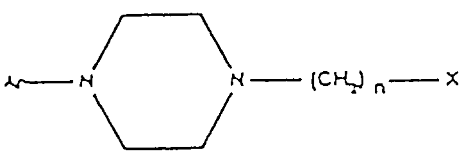

(II)

in der Ar und n die in Anspruch 1 angegebenen Bedeutungen haben und X für ein Halogenatom oder eine abspaltbare (austretende) Gruppe steht, ausgewählt aus Tosyloxy oder Mesyloxy,
mit einer Verbindung der allgemeinen Formel (III) umsetzt

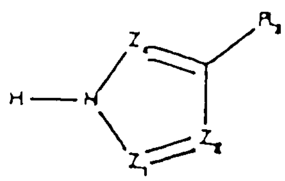

(III)

in der $Z_1$, $Z_2$, $Z_4$ und $R_3$ die in Anspruch 1 angegebenen Bedeutungen haben.

**4.** Verfahren zur Herstellung der Verbindungen nach einem der Ansprüche 1 und 2, dadurch gekennzeichnet, daß man eine Verbindung der allgemeinen Formel (IV)

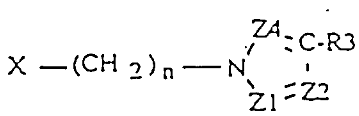

(IV)

in der $Z_1$, $Z_2$, $Z_4$, $R_3$, n und X die in den Ansprüchen 1 und 3 angegebenen Bedeutungen haben,

mit einer Verbindung der allgemeinen Formel (V) umsetzt

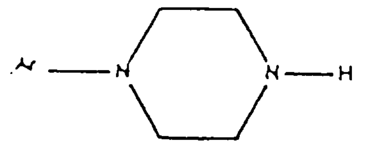 (V)

in der Ar die in Anspruch 1 angegebenen Bedeutungen hat.

5. Verfahren zur Herstellung der Verbindungen nach einem der Ansprüche 1 und 2, dadurch gekennzeichnet, daß man eine Verbindung der allgemeinen Formel (VI)

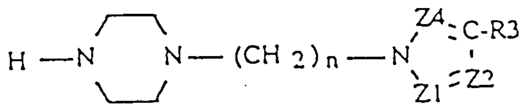 (VI)

in der $Z_1$, $Z_2$, $Z_4$, $R_3$ und n die in Anspruch 1 angegebenen Bedeutungen haben,
mit einer Verbindung der allgemeinen Formel (VII) umsetzt

Ar-X (VII)

in der Ar und X die in Anspruch 3 angegebenen Bedeutungen haben.

6. Verfahren zur Herstellung der Verbindungen nach einem der Ansprüche 1 und 2, dadurch gekennzeichnet, daß man eine Verbindung der allgemeinen Formel (VIII)

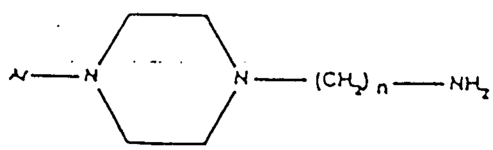 (VIII)

in der Ar und n die in Anspruch 1 angegebenen Bedeutungen haben, mit 2,5-Dimethoxytetrahydrofuran umsetzt.

7. Verfahren zur Herstellung der Verbindungen nach einem der Ansprüche 1 und 2, dadurch gekennzeichnet, daß man eine Verbindung der allgemeinen Formel (I), in der Ar, n, $Z_1$, $Z_2$, $Z_4$ und $R_3$ die in Anspruch 1 angegebenen Bedeutungen haben, in der jedoch mindestens einer der Substituenten $R_1$, $R_2$, $R_3$ und $R_4$ für einen Carboxylrest steht, mit einem Amin umsetzt.

8. Verfahren zur Herstellung der Verbindungen nach einem der Ansprüche 1 und 2, dadurch gekennzeichnet, daß man eine Verbindung der allgemeinen Formel (IX)

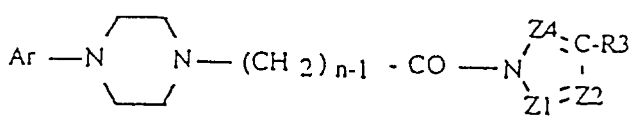 (IX)

in der Ar, $Z_1$, $Z_2$, $Z_4$, $R_3$ und n die in Anspruch 1 angegebenen Bedeutungen haben, reduziert.

9. Die Derivate nach einem der Ansprüche 1 oder 2 und ihre physiologisch akzeptablen Salze als Arzneimittel.

10. Verwendung der Derivate nach einem der Ansprüche 1 oder 2 und ihrer physiologisch akzeptablen Salze zur Herstellung eines Arzneimittels für die Behandlung der Angst, insbesondere zur Herstellung von Tranquilizern und/oder Anxiolytika, für die Behandlung der Depression, des Entzugssyndroms, von Bewußtseinsstörungen und der Hypertension.

11. Pharmazeutische Zusammensetzungen, dadurch gekennzeichnet, daß sie außer einem pharmazeutisch akzeptablen Träger mindestens ein Derivat nach einem der Ansprüche 1 oder 2 oder eines ihrer pharmazeutisch akzeptablen Salze enthalten.